**Europäisches Patentamt**

**European Patent Office**

**Office européen des brevets**

(19)

(11) Numéro de publication: **0 055 172**
**B1**

(12) # FASCICULE DE BREVET EUROPEEN

(45) Date de publication du fascicule du brevet:
**20.03.85**

(21) Numéro de dépôt: **81402006.1**

(22) Date de dépôt: **15.12.81**

(51) Int. Cl.⁴: **A 23 L 1/30,** A 23 D 5/04,
A 61 K 31/00 // C11B5/00,
A23J3/00

(54) **Composition à usage thérapeutique à base d'hydrolysats de protéines et de lipides riches en acides gras polyinsaturés.**

(30) Priorité: **18.12.80 FR 8026982**

(43) Date de publication de la demande:
**30.06.82 Bulletin 82/26**

(45) Mention de la délivrance du brevet:
**20.03.85 Bulletin 85/12**

(84) Etats contractants désignés:
**AT BE CH DE GB IT LI LU NL SE**

(56) Documents cités:
**BE - A - 770 604**
**FR - A - 2 280 323**
**FR - A - 2 436 178**
**US - A - 3 804 776**
**US - A - 3 849 554**

(73) Titulaire: **DISTRIVAL S.A., B.P. 67 20, rue Cachin,
F-50101 Cherbourg (FR)**

(72) Inventeur: **Chevalier, Jacques, 68, rue de la Charité,
F-69002 Lyon (FR)**
Inventeur: **Noel, Bernard, 24, rue Barbey d'Aurevilly,
F-50700 Valognes (FR)**

(74) Mandataire: **Phélip, Bruno et al, c/o Cabinet Harlé &
Phélip 21, rue de la Rochefoucauld, F-75009 Paris (FR)**

## Description

La présente invention concerne des compositions à base d'hydrolysats de protéines et de lipides riches en acides gras polyinsaturés et leur application à titre de produits diététiques et à la nutrition thérapeutique.

Les acides gras insaturés jouent un rôle essentiel dans le métabolisme général. Le mécanisme de leur action sur le catabolisme des lipoprotéines est bien connu. A cet effet, on peut mentionner les ouvrages récents ci-après:

– BROWN-M.S. GOLDSTEIN I.L. Receptor mediated control of Cholesterol metabolism, Science (1976) 191,150.

– FREDRICKSON D.S. BONNELLM, LEVY R.I., ERNST N. Dietary managment of hyperlipoproteinamia DHEW, Publication N) NIH, 75–110, National Heart and Lung Institute, Bethesda, Maryland.

D'autre part, avec la découverte de BERGSTROM et SAMUELSSON [Acta Chem. 1957, 11, p. 1086] sur la formation des prostaglandines à partir de certains acides gras poly-insaturés, l'apport à l'organisme d'acides gras comportant environ 18 à 24 atomes de carbone, précurseurs des prostaglandines, prend un intérêt de plus en plus évident.

Parmi les effets biologiques des prostaglandines actuellement reconnus, on peut citer notamment les effets sur les fonctions génitales, le système nerveux, les fonctions rénales, pulmonaires, cardiovasculaires, l'agrégation plaquettaire, comme l'ont montré les travaux de:

MONCADAS, CRYGLEWSKI R.I. BUNTINGS. VANE I.R., «An enzyme isolated from arteries transforms prostaglandin in diperoxides to an instable substance that inhibits plate agregation», Nature 1976, 263, 663–665.

R.O. VLES et U.M.T. HOUTSMULLER: «Connaissances actuelles sur les graisses alimentaires, leur importance en nutrition humaine» Rev. F2 des Corps Gras 24, 523–528 (1977).

ISAKSON P.C. RAS. A and NEEDLEMAN P. «Selective incorporation of 14 C arachidomic acid into the phospholipids of intact tissues and subsequent metabolism to 14 C prostaglandin» Prostaglandins, 12-739, 1976.

Les acides gras insaturés s'oxydent et se dégradent en quelques heures. Il en résulte que les acides gras insaturés doivent être utilisés pour la nutrition dans un délai très court après leur fabrication. Mais, même en respectant ce délai, ils sont en grande partie oxydés soit au niveau de leur fabrication, du stockage ou au cours de leur assimilation, soit au niveau gastrique et sont ainsi inactivés. Les acides gras insaturés sont en général fournis à l'organisme par l'intermédiaire de produits en contenant, tels que par exemple les huiles végétales, telles que tournesol, noix, maïs, soja, les huiles animales, telles que les huiles de poisson. Ces produits fragiles sont sensibles à la lumière, à l'oxydation, à l'action des sucs gastriques et ne sont que très partiellement métabolisables par l'organisme, en raison de la présence des acides gras insaturés qu'ils contiennent. Les vitamines liposolubles présentent sensiblement les mêmes caractéristiques.

Dans la suite de la présente description on désignera par «produit nutritionnel liposoluble dégradable» les produits choisis parmi les corps gras contenant les acides insaturés et les vitamines liposolubles ou leurs mélanges.

A titre d'autres documents de l'art antérieur concernant les huiles ou les vitamines on citera les documents ci-après:

– Le brevet US 3 804 776 qui concerne un procédé pour fabriquer des capsules contenant un ou plusieurs acides aminés, la capsulation étant réalisée à l'aide d'huile ou de graisse.

Ce procédé consiste à disperser l'acide aminé dans un mélange fondu d'huile et de graisse ayant un point de fusion supérieur à 40 °C et d'huile et de graisse ayant un point de fusion inférieur à 40 °C, et à verser la dispersion dans de l'eau à une température comprise entre 20 et 40 °C.

Les huiles et les graisses utilisées comme agent de capsulation permettent de protéger les acides aminés de l'environnement extérieur, tel que l'eau, les acides, les alcalis et les différentes enzymes.

Ce procédé consiste donc en un procédé de protection d'acides aminés ou polypeptides par des huiles ou des graisses; ce procédé permet l'obtention d'une phase lipophile continue sur les acides aminés à protéger.

– Le brevet US 3 849 554 qui concerne un procédé pour réduire le cholestérol dans le sérum sanguin. Ce procédé est en fait un procédé de traitement thérapeutique, qui consiste à administrer à l'homme, à titre de seule source d'aliment, une composition de régime constituée essentiellement de vitamines, de minéraux, d'une source d'acides aminés, d'une source d'acides gras et d'un hydrate de carbone, choisi parmi le glucose, le maltose, les polysaccharoses de glucose et leurs mélanges.

– Le brevet FR 79 23 149 publié sous le N° 2 436 178, qui concerne un procédé pour stabiliser les huiles et les graisses comestibles animales et végétales.

Ce procédé comprend les étapes suivantes:

a) le mélange d'une graisse ou d'une huile comestible avec une céréale ou avec une substance choisie parmi les amidons, les protéines et leurs mélanges;

b) le chauffage du mélange résultant à une température supérieure à 150 °C pour transformer l'huile ou graisse en un anti-oxydant;

c) le mélange d'une quantité efficace dudit anti-oxydant avec l'huile ou graisse animale ou végétale à stabiliser.

Ce procédé consiste à mélanger la graisse ou l'huile comestible avec par exemple une protéine, cependant il comporte une étape de chauffage à une température supérieure à 150 °C pour transformer l'huile en agent anti-oxydant. Il ne s'agit donc pas d'un traitement thermique modéré. Le traitement thermique selon ce procédé détruit totalement les effets thérapeutiques des acides gras insaturés.

On peut préciser que la stabilité recherchée dans le brevet FR 79 23 149 est uniquement une stabilité des huiles au rancissement et à la saponification, comme c'est le cas des huiles destinées à faire des fritures ou des assaisonnements. Il ne s'agit pas d'une stabilisation de l'activité thérapeutique des acides gras insaturés contenus dans certaines huiles végétales ou animales.

La demande de brevet FR 75 23 124 (publiée sous le N° 2 280 323) qui concerne un procédé de préparation de concentrés de protéines végétales, les produits ainsi obtenus et les aliments de remplacement du lait en contenant.

Ce procédé consiste à utiliser un mélange de produits résultant d'une part de l'hydrolyse acide de farine végétale et d'autre part de l'hydrolyse alcaline de ces mêmes farines végétales et à concentrer sous vide le mélange ci-dessus après neutralisation. Ce procédé est un procédé d'hydrolyse de protéines végétales dans le but d'obtenir un aliment de remplacement pour les veaux.

On remarquera que l'apport de protéines animales sous forme de farine de viande, de sang ou de poisson n'est fait que pour équilibrer la composition en acides aminés totaux. De même l'ajout de graisses n'est effectué que pour équilibrer la valeur énergétique de l'aliment par rapport au lait maternel.

Ce brevet ne concerne donc pas la stabilisation des acides gras insaturés contenus dans les huiles et leurs graisses et l'application de ces acides gras insaturés en thérapeutique.

– Le brevet BE 770 604 qui concerne un procédé pour l'obtention d'une poudre de vitamine E. Ce procédé consiste à upériser une émulsion aqueuse contenant un composé actif de vitamine E et de la gélatine hydrolysée ayant des caractéristiques particulières, définies dans la revendication 1 page 7 de ce brevet BE 770 604.

Ce procédé concerne donc la fabrication d'un produit pulvérulent contenant au moins 40% de vitamine E active. Les hydrolysats de protéines utilisés sont exclusivement des hydrolysats ayant un nombre de Bloom de zéro et un poids moléculaire compris entre 9000 et 11 000 et susceptible de se granuler ou de se comprimer.

Le mélange obtenu est séché par pulvérisation à des températures supérieures à 140 °C, puisqu'il s'agit d'une upérisation (supérieures à 177 °C voir page 3, 2ème paragraphe). Il faut noter par ailleurs que les agents conservateurs utilisés dans ce procédé sont des produits chimiques à des doses comprises entre 0,1 et 0,6%. Ce brevet ne concerne pas non plus la stabilisation de l'activité biologique des acides gras insaturés.

Aucun de ces documents ne concerne la stabilité de l'activité biologique des produits nutritionnels liposolubles, tels que les acides gras insaturés et les vitamines.

On a maintenant trouvé un procédé pour stabiliser l'activité biologique des produits nutritionnels liposolubles, qui permet l'obtention de produits ayant des propriétés nutritionnelles exceptionnelles, pouvant être conservés sans risque de détérioration et évitant une dégradation au niveau gastrique.

Sous sa forme la plus générale, le procédé selon la présente invention consiste:

a) à mélanger intimement une poudre d'hydrolysat de protéines qui n'a pas subi de traitement thermique supérieur à 85 °C, avec de 20 à 50% en poids par rapport à l'hydrolysat, d'huiles végétales ou animales comportant des acides gras insaturés, essentiels au métabolisme des prostaglandines, inaltérés, et éventuellement des vitamines liposolubles à une température comprise entre environ 20° et 55 °C, jusqu'à l'obtention d'une pâte homogène.

b) à conditionner la pâte ainsi obtenue.

Les hydrolysats de protéines sont connus pour leur valeur nutritionnelle. Ils sont obtenus par digestion enzymatique de protéines animales ou végétales en milieu acide ménagé. Après digestion enzymatique, les hydrolysats sont récupérés par des moyens classiques, tels que la filtration, la décantation et la centrifugation, de manière à obtenir une fraction soluble exempte de toute contamination bactériologique. De tels hydrolysats sont ensuite concentrés, à basse température, sous vide, en général jusqu'à l'obtention d'une teneur en matière séche comprise entre 60 et 70%. A titre d'exemple de procédé pour l'obtention d'hydrolysats de protéines on peut citer notamment le brevet FR 76 15 809, publié sous le N° 2 352 498, qui est relatif à l'obtention de concentrés de protéines de poissons.

En général, de tels hydrolysats ont une composition sur extrait sec qui est approximativement la suivante:

| | |
|---|---|
| Protéines | 82 à 88% |
| Matières minérales | 9 à 16% |
| Lipides | moins de 1% |

Les protéines comprennent de 15 à 35% d'acides aminés libres, des microprotéines et des oligo-peptides. Les traces de lipides sont le plus souvent constituées par des acides gras.

Ces hydrolysats présentent, dans la mesure où ils n'ont pas subi de traitement thermique supérieur à 85 °C, un potentiel d'activités biologiques important, tant protéolytique que lipolytique, qui se manifeste dès que le produit est placé dans des conditions favorables, par exemple élévation du pH ou abaissement de la concentration.

A titre d'exemples d'hydrolysats de protéines appropriés aux fins de l'invention, on peut notamment citer les hydrolysats de poissons, les hydrolysats de protéines animales, tels que pancréas, les hydrolysats de protéines végétales, tels que ceux de protéines de soja et les hydrolysats de lactoprotéines. Dans le procédé de l'invention, les hydrolysats de protéines sont avantageusement mis en œuvre sous une forme pulvérulente après déshydratation d'un concentré par atomisation à basse température ou par lyophilisation.

On a trouvé que, non seulement les hydrolysats de protéines permettent de stabiliser l'activité biologique des produits nutritionnels liposolubles, mais qu'ils contribuent également, par un effet de synergie, à l'obtention d'un produit ayant des qualités nutritionnelles exceptionnelles.

Tous les travaux réalisés à ce jour en nutrition, ainsi que les recommandations de la FAO, à partir d'huiles végétales, plus ou moins hydrogénées, font état d'un apport nécessaire de 10 à 20 g d'acides gras essentiels par jour pour obtenir un effet anti-agrégant au niveau des plaquettes sanguines (10 à 12% de l'énergie totale du régime alimentaire).

Or, il est maintenant bien connu que les prostaglandines, médiateurs chimiques des cellules, sont synthétisées à partir des acides gras stockés sous forme de phospholipides au niveau des membranes. La production totale des prostaglandines par un adulte mâle en bonne santé est de l'ordre de 0,1 mg en 24 heures. On constate donc que seule une infime partie des acides gras essentiels du régime alimentaire est activé au niveau du métabolisme des prostaglandines.

Par le procédé objet de la présente invention on a mis en évidence que l'on obtient avec des doses de 40 à 60 mg d'acides gras essentiels par jour des effets similaires non seulement au niveau cardio-vasculaire, mais aussi au niveau pulmonaire, système nerveux, une stimulation hormonale et un renforcement du pouvoir immunitaire.

L'ensemble de ces effets, reconnus comme dus à la synthèse des prostaglandines prouvent que les produits liposolubles stabilisés selon l'invention sont plus de 200 fois plus actifs au niveau de leur utilisation biologique que lorsqu'ils sont utilisés directement dans le régime alimentaire, sans stabilisation conforme à l'invention.

Sans vouloir pour autant se limiter à une quelconque théorie, on pense que les produits nutritionnels liposolubles sont enrobés par l'hydrolysat de protéines au cours du mélange avec celui-ci. On pense qu'il se forme une phase hydrophile continue qui enrobe les acides gras et qui les protège contre tout agent susceptible de les inactiver, tel que le suc gastrique par exemple, et que cette propriété de stabilisation de l'activité biologique peut être attribuée à la présence dans l'hydrolysat, d'un complexe sélénium-tocophérol ou à la présence de certaines microprotéines du type apoprotéine ou à l'activité enzymatique résiduelle de l'hydrolysat ou à l'ensemble de ces facteurs. Ainsi, selon le procédé de l'invention les produits nutritionnels liposolubles gardent leur intégralité et atteignent sans être danaturés l'endroit de l'organisme où ils sont métabolisables.

On a en effet constaté que l'on peut influer sur la propriété de stabilisation de l'activité biologique des hydrolysats en ajoutant au cours ou après hydrolyse des enzymes protéolytiques ou lipolytiques.

On utilise avantageusement aux fins de l'invention des hydrolysats de viscères de poissons, de lactoprotéines, ou des hydrolysats de levure, ou un mélange de ces hydrolysats, obtenus par hydrolyse acide, par voie enzymatique ou par autolyse, à des températures inférieures à 85 °C.

En général, on peut stabiliser, selon le procédé de l'invention l'activité biologique des produits nutritionnels liposolubles dégradables quelconques. A titre d'exemples, on peut citer notamment les huiles végétales ou animales, telles que les huiles de maïs, de tournesol, de pépins de raison, de noix, de soja, les huiles de poissons, par exemple de maquereaux, de cabillaud, les vitamines liposolubles, notamment celles de type A, $D_3$ et E. On peut également stabiliser selon le procédé de l'invention l'activité biologique d'un mélange de ces produits, par exemple un mélange d'huiles et de vitamines liposolubles. Ainsi on peut obtenir selon l'invention des produits nutritionnels liposolubles stables, ayant des compositions variées selon les besoins nutritionnels à satisfaire.

Les produits nutritionnels obtenus selon le procédé de l'invention peuvent par exemple constituer des aliments diététiques ou thérapeutiques contenant les acides gras essentiels nécessaires au métabolisme des prostaglandines par le corps humain.

Ces produits nutritionnels peuvent également être un mélange de produits contenant des acides gras insaturés, par exemple un mélange de lipides en combinaison avec des vitamines liposolubles ou des mélanges de telles vitamines en quantités adaptées aux besoins nutritionnels. Le procédé selon l'invention assure une parfaite conservation de l'activité des vitamines tout en augmentant leur coefficient d'assimilation.

Les produits nutritionnels liposolubles dégradables sont mis en œuvre dans le procédé de l'invention dans un délai très court après leur obtention, de manière à éviter toute dégradation. Par exemple on pourra utiliser une huile de maquereau, soit immédiatement après son extraction, soit dans un délai d'environ 24 heures après celle-ci. De même, on utilisera avantageusement les huiles de première pression.

La première étape du procédé de l'invention consiste donc à mélanger intimement au moins un produit nutritionnel liposoluble dégradable dans un hydrolysat de protéines. De façon avantageuse, on incorpore progressivement le produit nutritionnel liposoluble dégradable dans l'hydrolysat de protéines sous forme pulvérulente à une température comprise entre environ 20 et 55 °C. De manière à obtenir une pâte nutritionnelle homogène on malaxe continuellement l'hydrolysat et le produit nutritionnel liposoluble dégradable par des moyens classiques appropriés. Le mélange doit être effectué à une température d'au moins environ 20 °C. Cependant la température ne doit pas dépasser 55 °C environ.

L'indice de péroxyde dans le produit ainsi préparé peut s'élever en fonction de la température de mélange choisie et de la qualité des matières grasses utilisées jusqu'à 10 à 15 meq./kg de produit. Puis il doit s'abaisser rapidement dans les semaines qui suivent la préparation du produit jusqu'à une valeur de traces de façon à garantir l'activité des acides gras. En fonction du type des matières grasses utilisées on doit sélectionner la meilleure température de préparation pour que l'indice de péroxyde retrouve une valeur négligeable dans le temps.

4

La quantité de produit nutritionnel liposoluble dégradable à mettre en œuvre dépend de la nature de l'hydrolysat de protéines et de la valeur nutritionnelle du produit souhaite et est comprise entre 20 et 50% en poids par rapport à l'hydrolisat.

La seconde étape du procédé de l'invention consiste à conditionner le mélange obtenu pour le mettre sous une forme appropriée pour l'utilisation comme aliments diététiques ou thérapeutiques. Avantageusement on conditionne le mélange obtenu en capsules, gastrorésistantes ou non, selon des moyens classiques connus de l'homme de l'art. Ils peuvent également être conditionnés sous la forme de comprimés, de dragées, de capsules, de sondes injectables ou sous toutes autres formes qui permettent leur utilisation en nutrition thérapeutique. Les produits nutritionnels obtenus selon le procédé de l'invention peuvent être utilisés en diététique ou thérapeutique humaine ou animale.

Les produits nutritionnels obtenus selon l'invention peuvent en outre être associés à des produits actifs à des fins thérapeutiques particulières. Par exemple on peut associer le produit nutritionnel selon l'invention avec des germes de blé ou des médicaments homéopathiques. L'adjonction de ces produits actifs peut être réalisée au cours du procédé de fabrication du produit nutritionnel selon l'invention.

L'invention va être maintenant décrite plus en détail par les exemples illustratifs non limitatifs ci-après.

Exemple 1

On a utilisé comme agent stabilisant actif une poudre obtenue après lyophilisation d'un concentré d'hydrolysat enzymatique de foie de poisson de mer. La digestion enzymatique a été effectuée par les propres enzymes contenues dans les matières soumises au traitement et par des protéases du type papaïne. L'hydrolyse a été réalisée à une température comprise entre 50 et 60 °C et à un pH compris entre 4 et 5,5 pendant environ 6 heures. Après décantation, centrifugation et filtration l'hydrolysat a été concentré sous vide à une température inférieure à 75 °C jusqu'à une teneur en matière sèche comprise entre 60 et 70%. Le concentré a été ensuite lyophilisé dans un appareil classique puis mis en poudre avant conditionnement. Sa composition chimique était approximativement la suivante sur extrait sec:

| | |
|---|---|
| Azote | 14 dont azote aminé libre : 4,8 |
| Lipides | 1 |
| Matières minérales | 12 |

Dans un malaxeur de 100 litres à bain-marie muni d'un brasseur horizontal tournant à une vitesse comprise entre 40 et 60 tours à la minute, maintenu par une circulation d'eau chaude à une température comprise entre 40 et 55 °C, on a mis 50 kg de poudre d'hydrolysat, on y a incorporé progressivement 25 kg d'huile de maïs de première pression; après intégration complète de l'huile on a obtenu une pâte homogène qui a ensuite été conditionnée en capsules gastrorésistantes ou non d'un poids déterminé en fonction de la destination thérapeutique du produit, humaine ou animale.

La pâte homogène obtenue ci-dessus présentait juste après son obtention la composition pondérale suivante:

| | |
|---|---|
| Protides | 44% |
| Lipides | 26% (dont 70% en acides gras polyinsaturés) |
| Matières minérales | 12% |

Cette pâte a été conservée à la température ambiante sans subir de modification de l'indice de peroxyde des lipides.

Cette constatation montre que le produit obtenu selon l'invention ne se dégrade pas au cours du temps. Il faut noter que l'huile de maïs non traitée se dégrade très rapidement.

Exemple 2

On a traité, conformément au procédé de l'exemple 1, avec de l'hydrolysat de protéines de poisson, en poudre, un produit nutritionnel constitué d'huile de maïs et de lécithine auquel on avait ajouté des germes de blé. On a obtenu un produit nutritionnel ayant la composition pondérale suivante:

| | |
|---|---|
| – huile de maïs | 25% |
| – lécithine | 10% |
| – germes de blé | 5% |
| – hydrolysat de protéines de poisson | 60% |

Le produit obtenu était stable et pouvait être conservé à la température ambiante.

Exemple 3

Application du produit selon l'invention en alimentation animale

Dans ces essais nutritionnels on a utilisé un produit nutritionnel obtenu selon le mode opératoire décrit à l'exemple 1, à partir d'hydrolysat de protéines de poisson en poudre et d'huile de tournesol dans une proportion pondérale de 50/50. Le produit a été mis sous forme de gélules et administré aux animaux comme indiqué ci-après:

– chez les volailles ayant reçu par jour une gélule du produit défini ci-dessus on a constaté une diminution de 30% du poids des lipides; ces lipides étaient composés de plus de 30% d'acides gras insaturés alors que normalement les lipides des volailles contiennent moins de 8% en poids d'acides gras insaturés.

Les œufs produits par les cailles auxquelles a été administré le produit ci-dessus (1 gélule par jour) possèdent une quantité de cholestérol diminuée de 50% par rapport aux cailles n'ayant pas subi ce traitement.

– chez les bovins, les mêmes gélules (1 gélule de 1 gramme par jour) permettent d'obtenir au bout de 10 jours un lait dont la composition en matières protéiques et en matières grasses est modifiée. On a constaté en effet, une augmentation de 10% de protéines, leur concentration étant de l'ordre de 42 g/l alors qu'habituellement la teneur en protéines du lait est de 38 g/l environ. Par contre une diminution des lipides du lait d'environ 30% a été constatée.

L'étude des protéines du lait a permis de mettre en évidence une modification des micelles de la caséine, dont le diamètre est plus petit que dans la caséine d'animaux témoins.

La composition en acides gras insaturés des matières grasses de ce lait est passée de 3% (lait d'animaux non traités) à plus de 8% (lait d'animaux traités avec le produit selon l'invention).

De même on a constaté une augmentation de 15 à 30% de la production de lait chez des chèvres ayant reçu une gélule par jour pendant 10 ou 20 jours.

Exemple 4

On a préparé, conformément au procédé de l'exemple 1, avec de l'hydrolysat de protéines de poisson en poudre, un produit nutritionnel constitué d'huile de tournesol vierge dans les proportions pondérales suivantes:
– hydrolysat de poisson 20 kg
– huile de tournesol vierge 18,5 kg

La pâte obtenue a été ensuite conditionnée en capsules de 200 mg de produit actif contenant 60 mg d'acides gras insaturés totaux.

Ces capsules ont ensuite été administrées sous contrôle médical à raison d'une par jour dans différents centres hospitaliers qui ont pu constater son efficacité sur l'asthénie, une augmentation de la résistance à la fatigue, une amélioration sur le plan des tests psychométriques et en particulier dans le domaine de la mémoire.

On donnera ci-après deux comptes-rendus de l'utilisation de ces capsules en thérapeutique humaine.

Une première étude a été effectuée sur un groupe de 38 patients avec surveillance clinique et biologique régulière. Avec un recul moyen de 6 mois et des extrêmes de 14 mois on a constaté une parfaite tolérance du produit et une remarquable efficacité sur l'asthénie.

On a pu observer une augmentation de la résistance à la fatigue et de meilleures performances à des efforts précis sur bicyclette ergométrique et sur tapis roulant.

On a pu observer également des améliorations sur le plan des tests psychométriques et en particulier dans le domaine de la mémoire.

Une autre série d'essais a été effectuée avec le produit ci-dessus conditionné sous la forme de capsules gastrorésistantes. On a constaté une bonne tolérance du produit de l'invention. Les effets sur l'asthénie se sont révélés remarquables.

**Revendications pour les états contractants: BE, CH, DE, GB, IT, LI, LU, NL, SE.**

1. Composition à usage thérapeutique chez l'animal et l'homme pour améliorer les effets biologiques de la synthèse des prostaglandines et lutter contre l'asthénie, caractérisée en ce qu'elle contient un hydrolysat de protéines qui n'a pas subi de traitement thermique supérieur à 85 °C et, de 20 à 50% en poids par rapport à l'hydrolysat, d'huiles végétales ou animales comportant des acides gras insaturés essentiels au métabolisme des prostaglandines, inaltérés et qu'elle représente sous forme d'une pâte homogène résultant du mélange intime de la poudre d'hydrolysat de protéines sec avec les huiles à une température comprise entre 20 et 55 °C environ.

2. Composition selon la revendication 1, caractérisée en ce qu'elle comporte en outre des vitamines liposolubles.

3. Composition selon l'une des revendications 1 ou 2, caractérisée en ce que les huiles végétales ou animales sont des huiles de poisson, de maïs, de tournesol, de pépins de raisin, de noix, de soja, comportant des acides gras insaturés essentiels précurseurs des prostaglandines.

4. Composition selon l'une quelconque des revendications précédentes caractérisée en ce que les hydrolysats de protéines sont des hydrolysats de protéines animales, de poisson, de soja, de lactoprotéines, de levure ou leurs mélanges.

**Revendications pour l'état contractant: AT**

1. Procédé de préparation d'une composition pour usage thérapeutique chez l'animal ou l'homme pour améliorer les effets biologiques de la synthèse des prostaglandines et lutter contre l'asthénie caractérisé en ce qu'on mélange intimement à une température comprise entre 20 et 55 °C environ pour former une pâte homogène, une poudre d'hydrolysat de protéines qui n'a pas subi de traitement thermique supérieur à 85 °C, et de 20 à 50% en poids par rapport à l'hydrolysat, d'huiles végétales ou animales comportant des acides gras insaturés, essentiels au métabolisme des prostaglandines, inaltérés.

2. Procédé de préparation selon la revendication 1, caractérisé en ce qu'on ajoute au mélange des vitamines liposolubles.

3. Procédé de préparation selon l'une des revendications 1 ou 2, caractérisé en ce que les huiles végétales ou animales sont des huiles de poisson, de maïs, de tournesol, de pépins de raisin, de noix, de soja, comportant des acides gras insaturés essentiels précurseurs des prostaglandines.

4. Procédé de préparation selon l'une quelconque des revendications précédentes caractérisé en ce que les hydrolysats de protéines sont des hydrolysats de protéines animales, de poisson, de soja, de lactoprotéines, de levure ou leurs mélanges.

**Patentansprüche für die Vertragsstaaten: BE, CH, DE, GB, IT, LI, LU, NL, SE**

1. Zusammensetzung zur therapeutischen Verwendung bei Tier und Mensch, um die biologischen Effekte der Prostaglandin-Synthese zu verbessern und die Asthenie zu bekämpfen, dadurch gekennzeichnet, dass sie ein Protein-Hydrolysat, das keine Wärmebehandlung über 85 °C erfahren hat, und 20 bis 50 Gewichtsprozent, bezogen auf das Hydrolysat, pflanzliche oder tierische Öle enthält, die für den Metabolismus von Prostaglandinen essentielle, ungesättigte Fettsäuren unverändert aufweisen, und dass sie in Form einer homogenen Paste vorliegt, die beim gründlichen Mischen von trockenem Protein-Hydrolysat-Pulver mit den Ölen bei einer Temperatur von etwa 20 bis 55 °C entsteht.

2. Zusammensetzung nach Anspruch 1, dadurch gekennzeichnet, dass sie ausserdem fettlösliche Vitamine aufweist.

3. Zusammensetzung nach einem der Ansprüche 1 oder 2, dadurch gekennzeichnet, dass die pflanzlichen oder tierischen Öle Fisch-, Mais-, Sonnenblumen-, Traubenkern-, Nuss- oder Sojaöle sind, die ungesättigte Fettsäuren aufweisen, welche essentielle Vorstufen von Prostaglandinen sind.

4. Zusammensetzung nach irgendeinem der vorangehenden Ansprüche, dadurch gekennzeichnet, dass die Protein-Hydrolysate Hydrolysate von tierischen Proteinen, Fisch, Soja, Lactoproteinen, Hefe oder deren Mischungen sind.

### Patentansprüche für den Vertragsstaat: AT

1. Verfahren zur Herstellung einer Zusammensetzung für die therapeutische Verwendung bei Tier oder Mensch, um die biologischen Effekte der Prostaglandin-Synthese zu verbessern und die Asthenie zu bekämpfen, dadurch gekennzeichnet, dass man ein Protein-Hydrolysat-Pulver, das keine Wärmebehandlung über 85 °C erfahren hat, und 20 bis 50 Gewichtsprozent, bezogen auf das Hydrolysat, pflanzliche oder tierische Öle, die ungesättigte Fettsäuren, welche für den Metabolismus von Prostaglandinen essentiell sind, unverändert aufweisen, bei einer Temperatur von etwa 20 und 55 °C gründlich mischt, um eine homogene Paste zu bilden.

2. Verfahren nach Anspruch 1, dadurch gekennzeichnet, dass man die Mischung mit fettlöslichen Vitaminen versetzt.

3. Verfahren nach einem der Ansprüche 1 oder 2, dadurch gekennzeichnet, dass die pflanzlichen oder tierischen Öle Fisch-, Mais-, Sonnenblumen-, Traubenkern-, Nuss- oder Sojaöle sind, die ungesättigte Fettsäuren aufweisen, welche essentielle Vorstufen von Prostaglandinen sind.

4. Verfahren nach irgendeinem der vorangehenden Ansprüche, dadurch gekennzeichnet, dass die Protein-Hydrolysate Hydrolysate von tierischen Proteinen, Fisch, Soja, Lactoproteinen, Hefe oder deren Mischungen sind.

### Claims for the Contracting States: BE, CH, DE, GB, IT, LI, LU, NL, SE

1. An animal or human therapeutical composition for improvement of biological consequences of prostaglandin biosynthesis and for asthenia reduction, characterized in that it comprises a protein hydrolysate, which was not subjected to a temperature higher than 85 °C, and from 20% to 50% by weight, based on hydrolysate, of plant or animal oils comprising undamaged unsaturated fatty acids essential for prostaglandin metabolism and in that it is a homogeneous paste resulting from the throughout mixture of the dry protein hydrolysate powder and the oils at a temperature in the range from about 20 °C to about 55 °C.

2. A composition according to claim 1 characterized in that it comprises further liposoluble vitamins.

3. A composition according to claim 1 or to claim 2, characterized in that plant or animal oils are selected from fish, corn, sunflower, stone-grapes, walnut, soybean oils, comprising essential unsaturated fatty acids required for prostaglandin biosynthesis.

4. A composition according to any one of the preceding claims characterized in that protein hydrolysates are hydrolysates of animal, fish, soybean proteins, of milk proteins, of yeast or mixture thereof.

### Claims for the Contracting State: AT

1. A method for producing an animal or human therapeutical composition for improvement of biological consequences of prostaglandin biosynthesis and for asthenia reduction characterized in that protein hydrolysate powder, which was not subjected to a temperature higher than 85 °C, and from 20% to 50% by weight, based on hydrolysate, of animal or plant oils comprising unsaturated fatty acids essential for undamaged prostaglandin metabolism are throughoutly mixed at a temperature in the range from about 20 °C to about 55 °C to produce an homogeneous paste.

2. A method according to claim 1, characterized in that liposoluble vitamins are added to the mixture.

3. A method according to claim 1 or 2, characterized in that plant or animal oils are fish, corn, sunflower, stone-grapes, walnut, soybean oils, comprising essential unsaturated fatty acids required for prostaglandin biosynthesis.

4. A method according to any claim 1 to 3 characterized in that protein hydrolysates are hydrolysates of animal, fish, soybean proteins, of milk proteins, of yeast or of mixtures thereof.